# EUROPEAN PATENT SPECIFICATION

(11) **EP 1 112 500 B1**
(45) Date of publication and mention of the grant of the patent: **22.09.2004**
(21) Application number: 99968716.3
(22) Date of filing: 07.09.1999
(51) Int. Cl.: G01N 33/68, G01N 33/574

(54) **TAU AS A MARKER FOR EARLY CNS DAMAGE**
TAU ALS MARKER FÜR FRÜHE SCHÄDEN DES ZENTRALEN NERVENSYSTEMS
TAU UTILISE COMME MARQUEUR DANS LE DIAGNOSTIC PRECOCE DE LESIONS DU SYSTEME NERVEUX CENTRAL

(30) Priority: 08.09.1998 EP 98870190
(43) Date of publication of application: 04.07.2001
(73) Proprietor: INNOGENETICS N.V., 9052 Ghent (BE)
(72) Inventor: HULSTAERT, Frank, B-9050 Gentbrugge (BE); VANMECHELEN, Eugeen, B-9810 Nazareth-Eke (BE); VANDERSTICHELE, Hugo, B-9000 Ghent (BE); VAN DE VOORDE, André, B - 9160 Lokeren (BE); VAN GOOL, Stefaan, B - 3020 Herent (BE)
(86) International application number: PCT/EP1999/006592
(87) International publication number: WO 2000/014546

(56) References cited:
- WO-A-94/13795
- WO-A-96/04309
- SHOJI M ET AL: "Combination assay of CSF Tau, A-beta-1-40 and A-beta-1-42(43) as a biochemical marker of Alzheimer's disease." JOURNAL OF THE NEUROLOGICAL SCIENCES 158 (2). 134-140. ISSN: 0022-510X, 30 June 1998 (1998-06-30), XP002083581
- NISHIMURA T ET AL: "Basic and clinical studies on the measurement of tau protein in cerebrospinal fluid as a biological marker for Alzheimer's disease and related disorders: Multicenter study in Japan." METHODS AND FINDINGS IN EXPERIMENTAL AND CLINICAL PHARMACOLOGY 20 (3). 227-235. ISSN: 0379-0355,April 1998 (1998-04), XP002083582
- TRANCHANT C: "Tau proteins and neurodegenerative diseases" M/S MEDECINE SCIENCES, vol. 13, no. 8/09, August 1997 (1997-08), pages 989-997, XP002083585

## Description

### FIELD OF THE INVENTION

The present invention relates to the field of CNS damage. The present invention relates to a new method for the early diagnosis of CNS damage by detection and/or quantification of tau.

### BACKGROUND OF THE INVENTION

Damage of the central nervous system (CNS damage) is caused by various inducing agents among which different disease processes, physical or chemical agents, anoxia and ischemia. Disease processes include space occupying lesions, invasion or metastasis of the brain caused by different malignancies and/or infection by a number of organisms.

Tumors of the CNS, may originate locally (primary tumors) or may spread to the CNS (metastases). Primary tumors arise from glial cells (astrocytoma, oligodendroglioma, glioblastoma), ependymal cells (ependymoma) or supporting tissue (meningioma, schwannoma, papilloma of the choroid plexus). In childhood, tumors arise from more primitive cells (medulloblastoma, neuroblastoma, chordoma). Malignant astrocytoma or glioblastoma is the most common type of primary tumor in adults over age 20. Both benign and malignant primary CNS tumors are capable of producing neurologic impairment.

Leukemia is the most common type of cancer in children. During the last twenty years, the survival of children with leukemia has improved markedly based on the routine use of intensive chemotherapy alone or as combined treatment (radiotherapy and chemotherapy). Currently, the estimated overall 10-year survival rate is around 75%. Given the increasing number of childhood leukemia survivors, concern has arisen about long-term effects of anti-cancer chemotherapy and/or radiotherapy resulting in possible damage to the central nervous system and the need for an early quantitative determination of this CNS damage is increasing.

Bacterial meningitis may be defined as an inflammation in response to bacterial infection of the pia-arachnoid and the fluid residing in the space which it encloses and also of the fluid in the ventricles of the brain. The incidence of bacterial meningitis is between 4.6 and 10 cases per 100000 persons per year. *H. influenzae* is the most frequent cause, followed by *N. meningitidis* and *S. pneumoniae*. Once developed, characteristic features of bacterial meningitis include an increase in intracranial pressure, disruption of the blood-brain barrier, cerebral edema, and alterations in cerebral blood flow. The longer the duration of meningitis and the less effective the treatment, the greater the chances that complications and neurologic residua will develop. Approximately 10 percent of infants and children who have bacterial meningitis will be left with persistent unilateral or bilateral sensory hearing loss. Approximately 30 percent of children who have had bacterial meningitis later will turn out to have subtle learning deficits (Wilson et al., 1991).

Viruses can also affect the central nervous system in a variety of ways resulting in a distinction between viral meningitis, viral encephalitis, myelitis and CNS diseases due to slow virus infection. Other conditions that may cause CNS damage are chemical agents such as pharmaceuticals, chemotherapy or exposure to chemical compounds, and physical agents. Head injuries are frequent in industrialised countries, affecting many patients in the prime of life. To appreciate the medical and social magnitude of this problem it needs only to be recognised that almost 10 million Americans have head injuries yearly, about 20 percent serious enough to cause brain damage.

Another cause for CNS damage may be anoxia or ischemia. Anoxic-ischemic encephalopaty is a common and often disastrous condition, caused by a lack of oxygen to the brain, resulting from hypotension or respiratory failure. Acute ischemic stroke causes neuronal damage and is a major cause of neurological handicap in western society. Perinatal asphyxia may be associated with CNS damage as well. To date, clinical, electroencephalographic and neuroradiologic evaluation, together with cerebral blood flow studies are the most readily available methods. However, early and accurate evaluation of the severity of brain damage after a hypoxic-ischemic event, remains one of the most difficult problems in neonatal care.

For the detection of CNS invasion in leukemic children current diagnostic procedures include lumbar puncture, eye fundoscopy and brain imaging (Raichle, 1998). However, these diagnostic methods only allow detection of the CNS damage in a more advanced stage while already ongoing CNS damage in the early stages may be missed by these methods. Therefore, there is a need for additional diagnostic methods that allow early detection of CNS damage.

A number of neurological markers have recently become available which reflect conditions of the central nervous system, relating to cell death, axon growth/re-induction, inflammation and/or blood-brain barrier dysfunction. The microtubule-associated protein tau exists in different isoforms, of which 4 to 6 are found in adult brain but only 1 isoform is detected in fetal brain. The diversity of the isoforms is generated from a single gene on human chromosome 17 by alternative mRNA splicing (Himmler, 1989; Goedert et al., 1989; Andreadis et al., 1992). The most striking feature of tau protein, as deduced from molecular cloning, is a stretch of 31 or 32 amino acids, occurring in the carboxy-terminal part of the molecule, which can be repeated either 3 or 4 times. Additional diversity is generated through 29 or 58 amino acid-long insertions in the NH₂-terminal part of tau molecules (Goedert et al., 1989). In vivo tau promotes microtubule assembly and stability in the axonal compartment of neurons by interactions involving its microtubule binding domain which is localised in the repeat region of tau (255-381) (Lewis et al., 1988). In normal circumstances adult brain contains 2 - 3 mol phosphate per mole of tau (Selden and Pollard, 1983; Ksiezak-Reding et al., 1992). Phosphorylation of different sites in normal tau as studied in rat and humans is dependent on the developmental state (Lee et al., 1991; Bramblett et al., 1993; Goedert et al., 1993). Tau variants of 60, 64 and 68 kDa arising as a consequence of phosphorylation have been detected in brain areas showing neurofibrillary tangles (Delacourte et al., 1990; Goedert et al., 1992; Flament and Delacourte, 1990, Greenberg and Davies, 1990). These brains contain 6-8 mol phosphate per mol tau (Ksiezak-Reding et al., 1992). In tau isolated from paired helical filaments, phosphorylation can occur at several positions (Iqbal et al., 1989; Lee et al., 1991; Hasegawa et al., 1992). Detection of normally and abnormally phosphorylated tau in brain extracts is done either via antibodies (Mab Alz50: Ghanbari et al., 1990; Mab Ab423: Harrington et al., 1991; Mab AT120 : Vandermeeren et al., 1993; Mab AT180; Mab AT270 : International application published under WO 95/17429 and Mab AT8 : International application published under WO 93/08302), or via the change in molecular weight (Flament and Delacourte, 1990), or else by functional assay (Bramblett et al., 1992). A combination of monoclonal antibodies, each recognising specific epitopes of tau, has been used to detect the presence of normally and abnormally phosphorylated tau in CSF (Van de Voorde et al., 1995). Tau has been used as a marker to discriminate dementia with altered cytoskeletal properties such as Alzheimer's disease from normal aged subjects or from patients with other types of dementia.

### AIMS OF THE INVENTION

It is the aim of the present invention to provide
1. a method for the in vitro detection and/or quantification of CNS damage in an individual before said CNS damage is detectable by standard diagnostic methods based on brain imaging, lumbar puncture or eye fundoscopy, said CNS damage being caused by a primary brain tumor, benign or malignant, brain metastasis, parasite derived cysts, hydrocephalus and/or subdural haematoma, by invasion or metastasis of the CNS, by organisms, by anoxia or ischemia, by a chemical agent which is gene therapy, pharmaceuticals, chemotherapy or exposure to chemical compounds, by physical agents or by a combination of these mechanisms, said method comprising the step of determining the level of tau in a CSF sample from said individual and comparing it to the level of tau in a CSF sample from control healthy individuals, whereby a level of tau above a cut-off value, determined based on the level of tau in a CSF sample from control healthy individuals, is an indication of the individual having brain damage.

It is a more specific aim of the present invention to provide a method for the early detection and/or quantification of CNS damage in an individual, said CNS damage being caused by space-occupying lesions of the CNS, by invasion or metastasis of the CNS, by organisms, by anoxia or ischemia, by chemical agents, by physical agents, or by a combination of these mechanisms.

It is a more specific aim of the present invention to provide a method for the early detection and/or quantification of CNS damage in an individual, said CNS damage being caused a primary brain tumour, benign or malignant, brain metastasis or a subdural haematoma.

It is another more specific aim of the present invention to provide a method for the early detection and/or quantification of CNS damage in an individual, said CNS damage being caused by invasion of the CNS by leukemia, lymphoma or breast cancer.

It is another more specific aim of the present invention to provide a method for the early detection and/or quantification of CNS damage in an individual, said CNS damage being caused by bacteria or viruses causing encephalitis or meningitis.

It is another more specific aim of the present invention to provide a method for the early detection and/or quantification of CNS damage in an individual, said CNS damage being caused by stroke, by cerebral infarction, by cerebral haemorrhage, by thrombosis, by perinatal asphyxia, by Binswanger disease or by vasculitis.

It is another more specific aim of the present invention to provide a method for the early detection and/or quantification of CNS damage in an individual, said CNS damage being caused by chemotherapy.

It is another more specific aim of the present invention to provide a method for the early detection and/or quantification of CNS damage in an individual, said CNS damage being caused by trauma, stroke, intracranial pressure or radiation.

It is another aim of the present invention to provide a method for the early detection and/or quantification of CNS damage in an individual, said CNS damage being caused by space-occupying lesions of the CNS, by invasion or metastasis of the CNS, by organisms, by anoxia or ischemia, by chemical agents, by physical agents, or by a combination of these mechanisms in order to evaluate the effect of treatment on said CNS damage.

It is another aim of the present invention to provide a method to screen or monitor the effect of compounds which prevent or treat CNS damage.

All the aims of the present invention are considered to be met by the embodiments as set out below.

### DETAILED DESCRIPTION OF THE INVENTION

The present invention relates to a method for the early detection and/or quantification of CNS damage in an individual, said CNS damage being caused by space-occupying lesions of the CNS, by invasion of the CNS, by organisms, by anoxia or ischemia, by chemical agents, by physical agents or by a combination of these mechanisms. This method comprises the step of determining and/or quantifying the level of tau in an individual and comparing it to the level of tau in control healthy individuals.

The present invention relates to the surprising finding that tau levels in CSF samples from children with leukemia are increased compared to upper limit values for healthy individuals. These increased tau levels are an indication of latent central nervous system invasion and already ongoing CNS damage long before this CNS damage can be measured by the current diagnostic procedures. Also in individuals that were suffering space occupying lesions of the CNS, invasion or metastasis of the CNS, ischemia, stroke or meningitis, increased tau levels were observed in an early stage. Accordingly, tau can be used as an aspecific marker for the early detection of CNS damage caused by invasion of the CNS by leukemia and in general, as an aspecific marker for the early detection of CNS damage caused by CNS damaging agents such as space-occupying lesion of the CNS, invasion or metastasis of the CNS, organisms, anoxia or ischemia, chemical agents, physical agents, or a combination of these mechanisms.

The central nervous system (CNS) is that part of the nervous system which, in vertebrates, consists of the brain and spinal cord, to which sensory impulses are transmitted and from which motor impulses pass out, and which supervises and co-ordinates the activity of the entire nervous system.

The term "CNS damage" refers to any condition of the CNS which is associated with a neuronal malfunctioning and which is caused by a specific inducing agent or damaging agent. More specifically, CNS damage refers to disease processes that include but are not limited to space-occupying lesions of the CNS, invasion or metastasis of the CNS and/or organisms. Space occupying lesions may be, for example, primary brain tumours, benign or malignant, brain metastasis, parasite derived cysts such as *Taenia solium* or *Echinococcus granulosus*, hydrocephalus and/or subdural haematoma. Invasion or metastasis of the CNS can be caused by malignancies such as leukemia, lymphoma, breast cancer, lung cancer, melanoma and/or gastrointestinal malignancies or other types of cancers. Organisms that may infect the CNS and cause CNS damage include but are not limited to prions, viruses, bacteria or parasites. Bacteria and viruses that infect the CNS may cause meningitis, encephalitis, neuro aids or neuroborreliose.

They include but are not limited to *Neisseria meningitidis, H. influenzae, S. pneumoniae, Herpes simplex meningoencephalitis* and *Herpes simplex gluteolis*. CNS damage can also be caused by anoxia or ischemia during anestesia, perinatal asphyxia, drowning, asthma, stroke, cerebral infarction, thrombosis, cerebral haemorrhage, CO poisoning, Binswanger disease and/or vasculitis. Chemical agents causing CNS damage include but are not limited to gene therapy, pharmaceuticals, chemotherapy and exposure to chemical compounds. CNS damage can also be caused by physical agents such as trauma, radiation, hypothermia, hyperthermia, intracranial pressure or stroke. It is also possible that more than one of the above mentioned causing agents are responsible for the CNS damage.

The present invention thus provides a method for the early detection and/or quantification of said CNS damage by determining the level of tau. "Early detection and/or quantification of CNS damage" means that the CNS damage is determined by a method that allows it to be detected before it is detectable by the current methods.

The term "tau" as referred to in the present application can be any form of tau, including any state of phosphorylation. The level of tau is determined qualitatively and/or quantitatively as a measure for the degree of CNS damage. Tau can be detected in vitro as well as in vivo.

The method for the early in vitro detection of CNS damage in an individual comprises the steps of obtaining a sample from said individual, determining and/or quantifying the level of tau in said sample and comparing it to the level of tau in a sample of control healthy individuals. The term "sample" refers to any source of biological material, for instance body fluids, hair, epithelial cells, peripheral blood or any other sample comprising tau protein. In a preferred embodiment, tau can be detected and/or quantified in vitro by analysis of the level of tau in a body fluid sample of the patient. The term "body fluid" refers to all fluids that are present in the human body including but not limited to blood, lymph, urine and cerebrospinal fluid (CSF). In a more specific embodiment of the present invention tau is detected and/or quantified in a cerebrospinal fluid sample taken from the patient. In another specific embodiment of the invention tau is detected and/or quantified in a sample of blood derivatives of the patient. The blood sample can include the whole sample as taken from the patient. More preferably the blood sample includes a plasma sample or a serum sample.

Tau can be detected and/or quantified by any method known, including but not limited to the use of antibodies, the change in molecular weight (Flament and Delacourte, 1990), or else by functional assay (Bramblett et al., 1992). In a preferred embodiment tau can be detected by an immunoassay comprising at least the following steps:
- obtaining a sample from the patient; and
- bringing said sample into contact with a monoclonal antibody (primary antibody or capturing antibody) recognising tau, under conditions being suitable for producing an antigen-antibody complex; and
- detecting the immunological binding of said antibody to said sample.

Advantageously, the monoclonal antibody used in the invention is in an immobilised state on a suitable support. Alternatively, the present process may be put into practice by using any other immunoassay format known to the person skilled in the art.

The process for the detection of the antigen can then be carried out by bringing together said antigen-antibody complex formed by the antigen and the primary antibody recognising tau with:
a) a secondary antibody (or detector antibody)
   * which can be a monoclonal antibody recognising an epitope of the tau-primary antibody complex but not recognising the primary antibody alone, or
   * which can be a polyclonal antibody recognising an epitope of the tau-primary antibody complex but not recognising the primary antibody alone, with said polyclonal antibody being preferably purified by immunoaffinity chromatography using immobilised tau or the tau-primary antibody complex.
b) a marker either for specific tagging or coupling with said secondary antibody, with said marker being any possible marker known to the person skilled in the art;
c) appropriate buffer solutions for carrying out the immunological reaction between the primary antibody and the sample, between the secondary antibody and the tau-primary antibody complex and/or between the bound secondary antibody and the marker, and,
d) possibly also, for standardisation purposes, a purified protein or synthetic peptide reactive with the antibodies that recognise tau.

Advantageously, the secondary antibody itself carries a marker or a group for direct or indirect coupling with a marker.

The term "epitope" refers to that portion of the antigen-antibody complex that is specifically bound by an antibody-combining site. Epitopes may be determined by any of the techniques known in the art or may be predicted by a variety of computer prediction models known in the art.

The expression "recognising", "reacting with", "immunological binding" or "producing an antigen-antibody complex" as used in the present invention is to be interpreted that binding between the antigen and antibody occurs under all conditions that respect the immunological properties of the antibody and the antigen.

Any monoclonal or polyclonal antibody that specifically recognises tau may be used for the detection of tau. Antibodies specifically recognising normally and/or abnormally phosphorylated tau include Alz50 (Ghanbari et al., 1990), Ab423 (Harrington et al., 1991), AT8 (International application published under WO 93/08302), AT120 (Vandermeeren et al., 1993); AT180 and AT270 (International application published under WO 95/17429) and AT100 (International application published under WO 96/04309). But also other antibodies known in the art that specifically recognise tau can be used.

The method for the early in vitro detection and/or quantification of CNS damage in an individual can also be used to evaluate the effect of a certain treatment on the CNS damage in said individual. Possible treatments that might influence the status of the CNS include but are not limited to drug treatments, chemotherapy, physical therapy, including radiotherapy and gene therapy.

The method for the early in vivo detection and/or quantification of CNS damage in an individual comprises the steps of determining and/or quantifying the level of tau in said individual and comparing it to the level of tau in control healthy individuals. In a preferred embodiment, tau can be detected in vivo by in vivo imaging. Tau can be visualised in situ by non-invasive methods including but not limited to brain imaging methods described by Arbit et al. (1995), Tamada et al. (1995), Wakabayashi et al. (1995), Huang et al. (1996), Sandrock et al. (1996), Mariani et al. (1997). These in vivo imaging methods may allow the localisation and visualisation of tau, for example, by use of labelled antibodies recognising tau.

Tau can also be used as an aspecific marker for in vivo imaging to evaluate the effect of a certain treatment on the CNS damage in an individual. Possible treatments that might influence the status of the CNS include but are not limited to drug treatments, chemotherapy, physical therapy, including radiotherapy and gene therapy.

Any kit that provides a tool for the detection of tau can be used for the diagnosis of the above-mentioned CNS damage.

A preferred kit for the in vitro diagnosis in an individual of CNS damage caused by space-occupying lesions of the CNS, by invasion or metastasis of the CNS, by organisms, by anoxia or ischemia, by chemical agents, by physical agents, or by a combination of these mechanisms is based on an immunoassay and comprises:
- at least a monoclonal antibody (primary antibody) which forms an immunological complex with an epitope of the tau protein;
- a secondary antibody
   * which can be a monoclonal antibody recognising an epitope of the tau-primary antibody complex but not recognising the primary antibody alone, or
   * which can be a polyclonal antibody recognising an epitope of the tau-primary antibody complex but not recognising the primary antibody alone, with said polyclonal antibody being preferably purified by immunoaffinity chromatography using immobilised tau protein or immobilised tau-primary antibody complex;
- a marker either for specific tagging or coupling with said secondary antibody;
- appropriate buffer solutions for carrying out the immunological reaction between the primary antibody and a test sample, between the secondary antibody and the tau-primary antibody complex, and/or between the bound secondary antibody and the marker;
- possibly, for standardisation purposes, a purified protein or synthetic peptide containing one of more tau epitopes.

The present invention also relates to a method to screen or monitor the effect of compounds which prevent or treat CNS damage comprising the step of determining the level of tau and comparing it to the level of tau in a control sample.

The present invention will now be illustrated by reference to the following examples that set forth particularly advantageous embodiments. However, it should be noted that these examples are illustrative and can not be construed as to restrict the invention in any way.

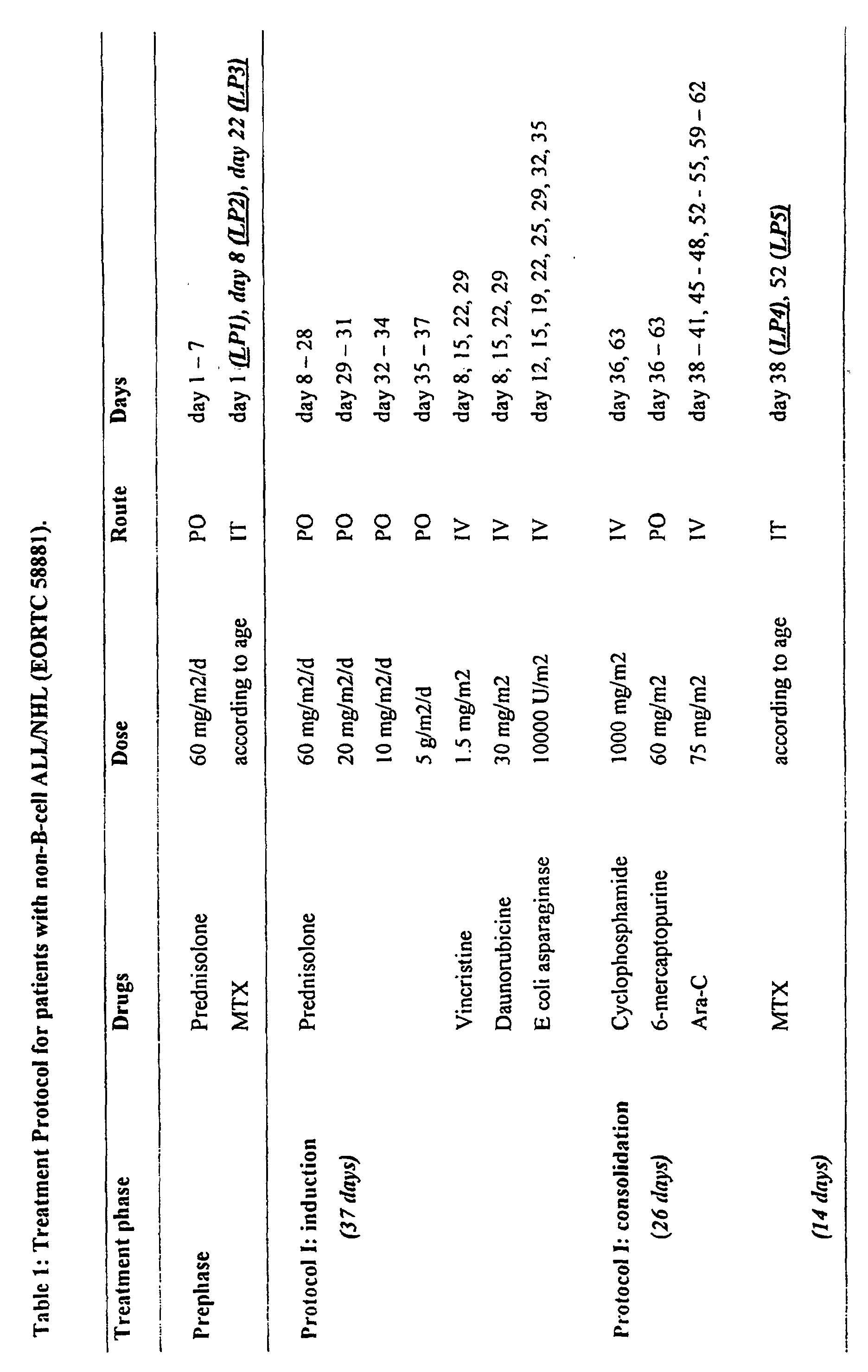

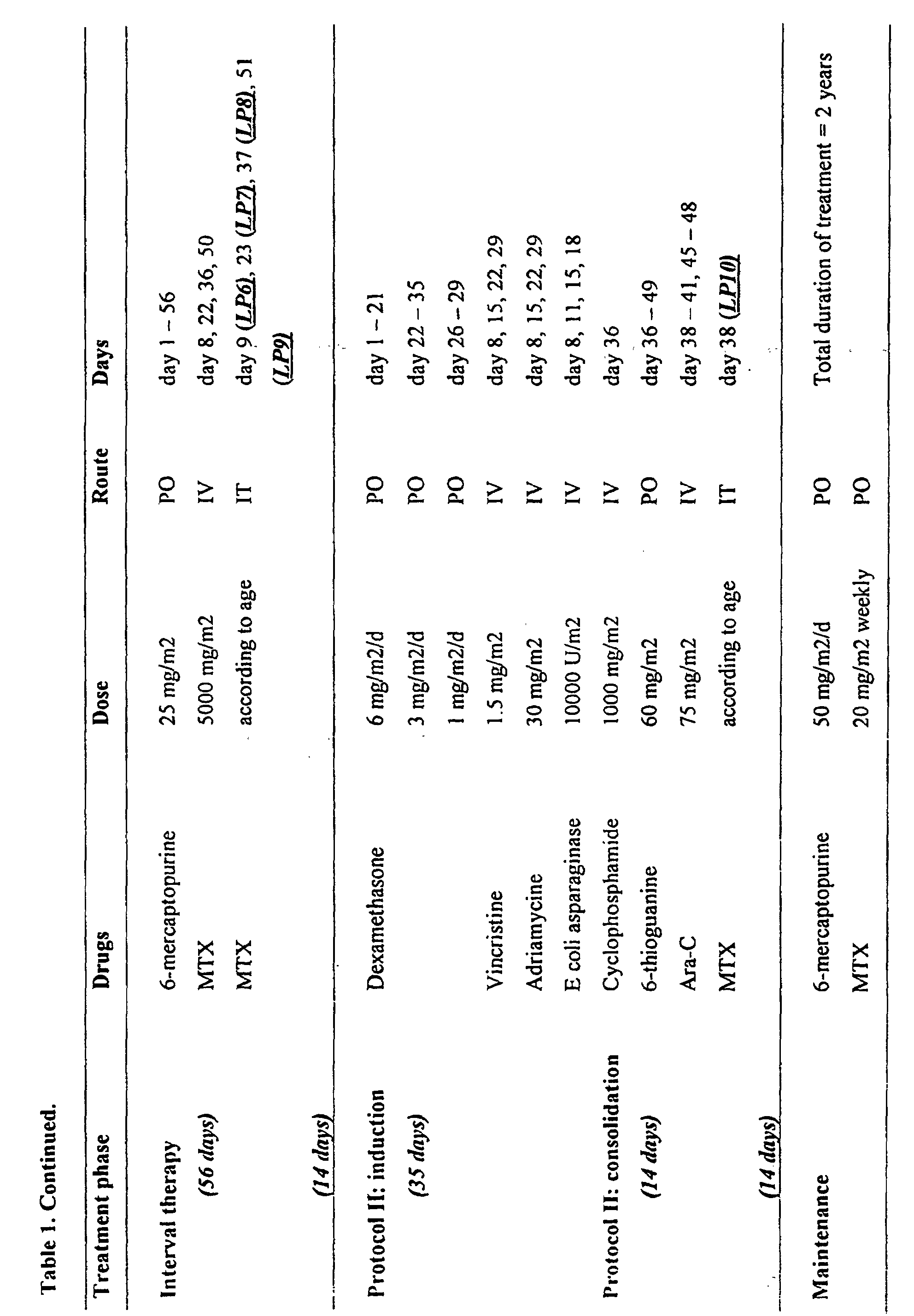

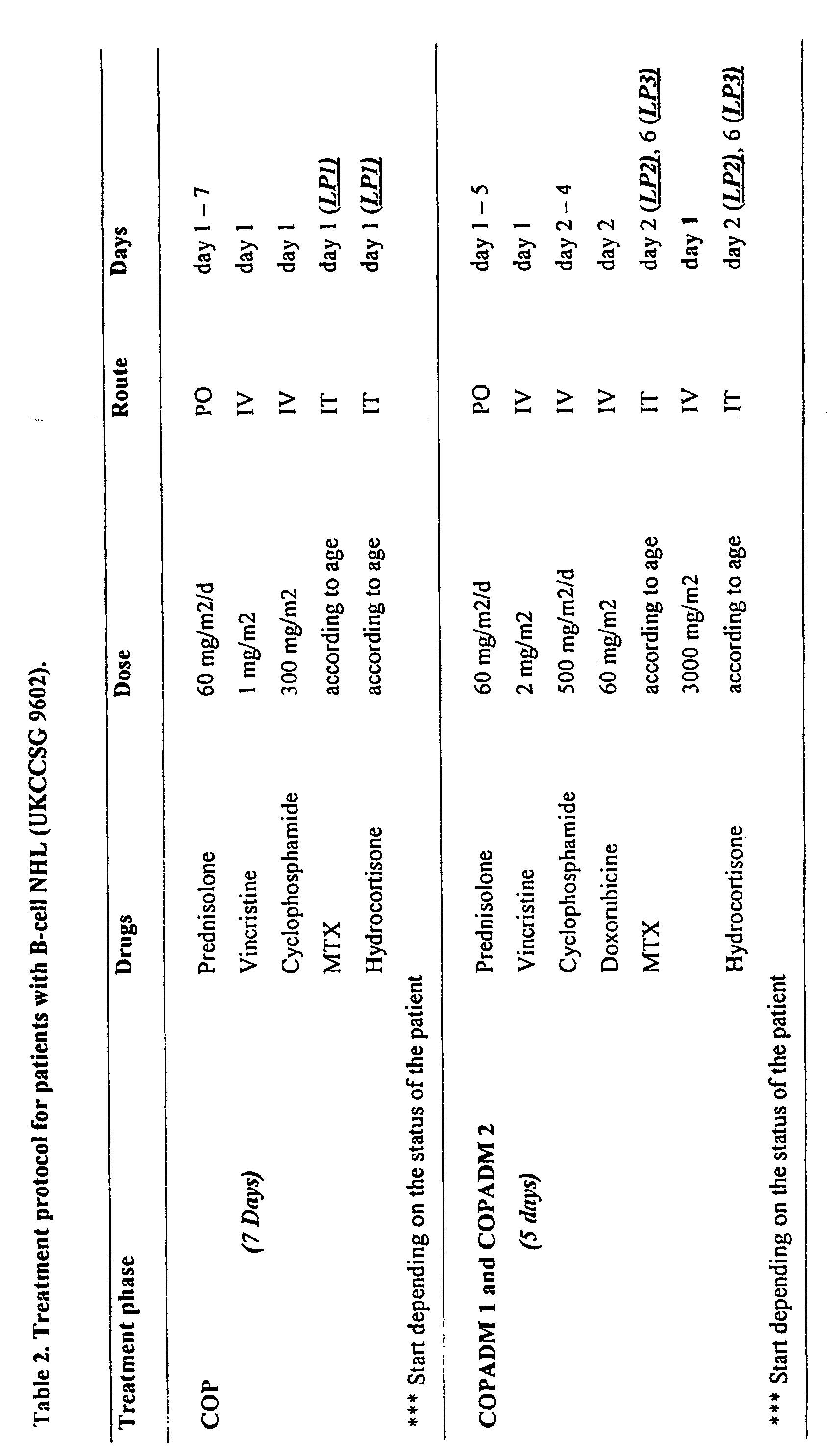

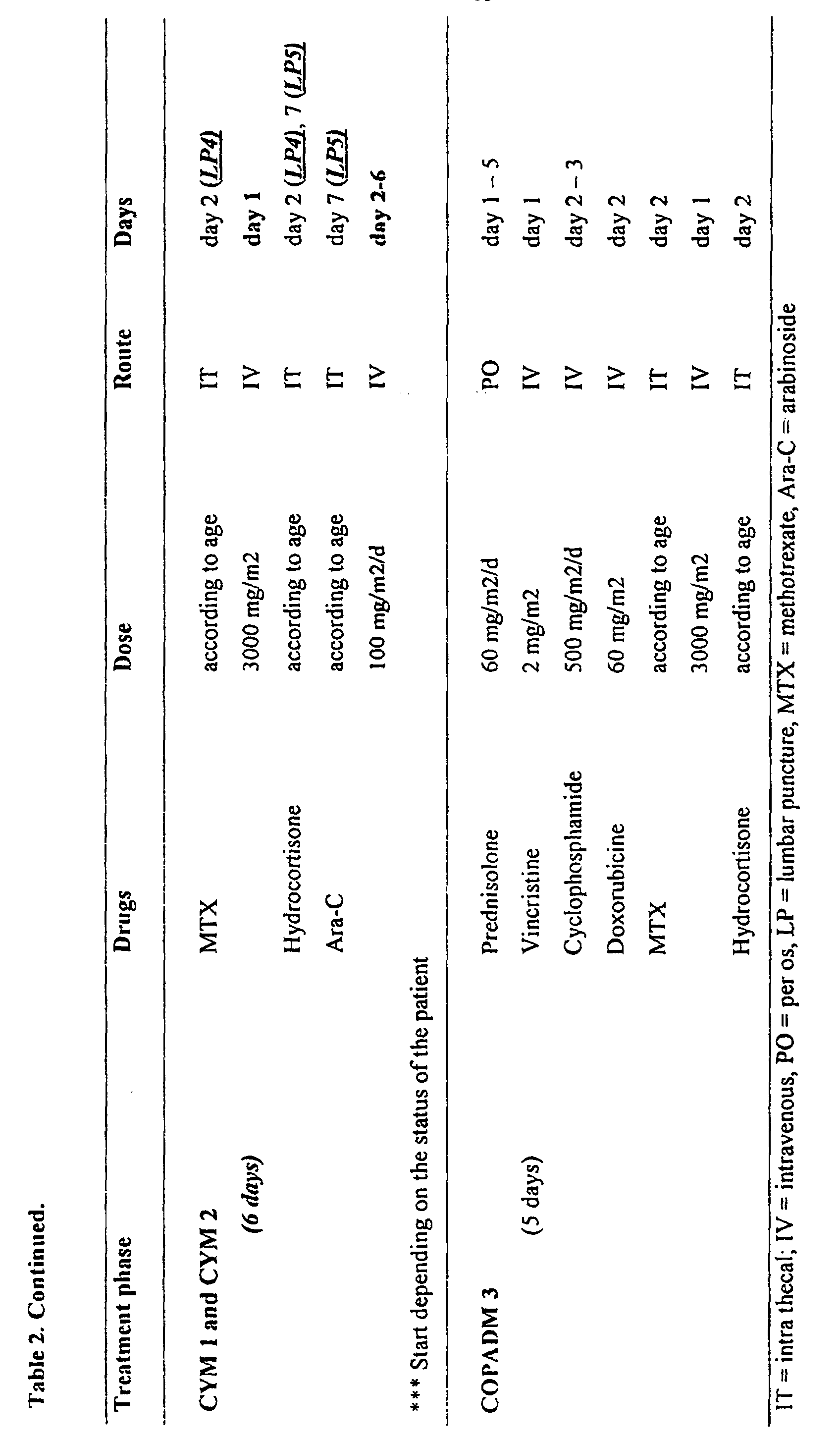

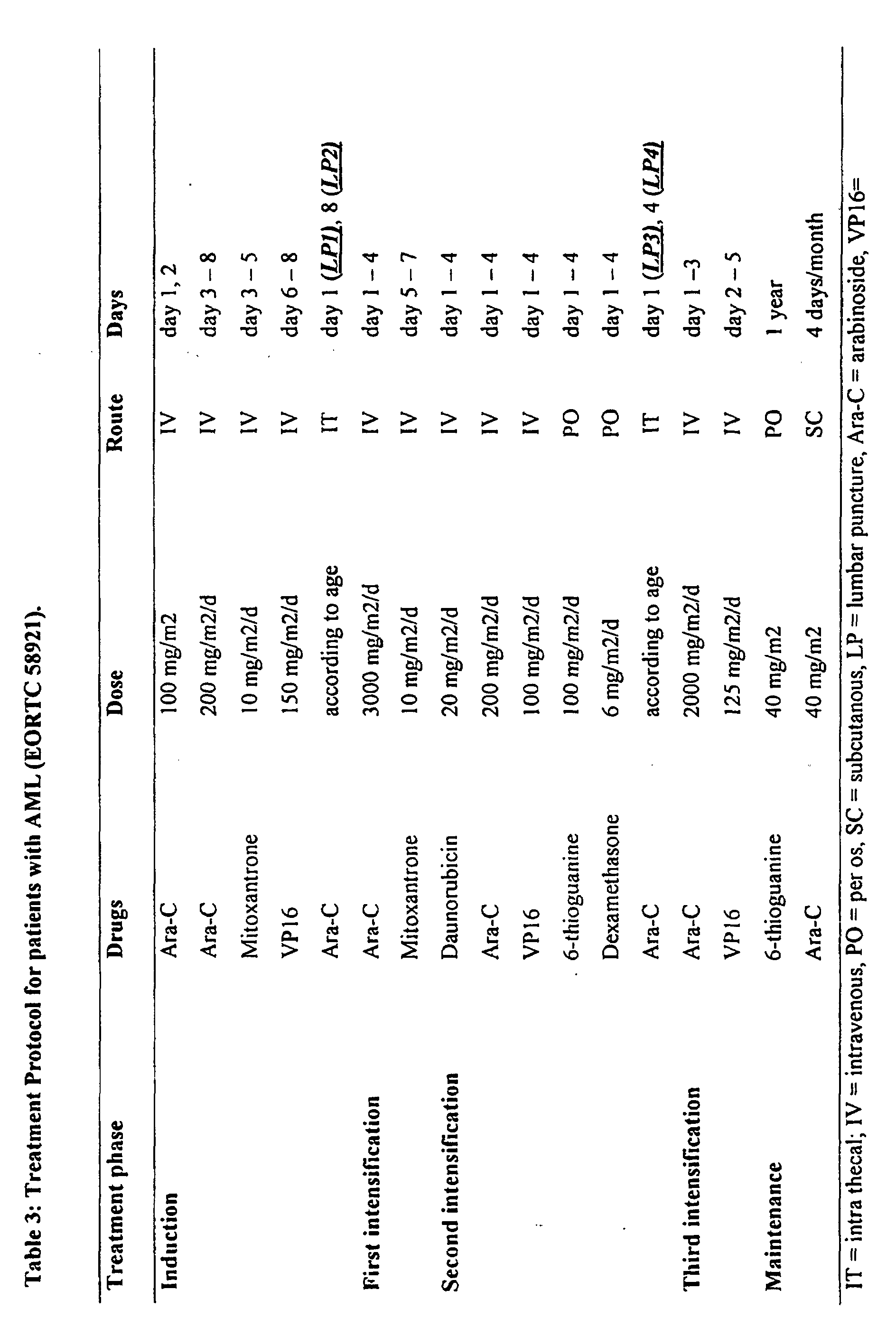

**Table 4.**

| **Level of tau in CSF samples of patients with possible CNS damage caused by different factors.** | | | | | |
|---|---|---|---|---|---|
| **Cause of CNS damage**^{**c**} | **Centre**^{**a**} | **Patient** | **Age** | **Sex**^{**b**} | **Tau level (pg/ml)** |
| **SPACE OCCUPYING LESIONS, INVASION OR METASTASIS** | | | | | |
| Subdural haematome | 01 | 025 | 39 | M | 263 |
| Anaplastic oligoastrocytoma | 01 | 032 | 68 | M | 329 |
| Cerebral metastasis | 08 | 024 | 70 | F | 292 |
| Oligodendroglioma WHO III | 08 | 031 | 48 | M | 335 |
| Metastasis of breast cancer | 08 | 020 | 52 | F | 150 |
| | | | | | |

| **BLEEDING, INFARCTION OR ISCHEMIA** | | | | | |
|---|---|---|---|---|---|
| Stroke - CVA | 01 | 021 | 62 | F | 451 |
| Multiple lacunar stroke | 01 | 026 | 56 | F | 250 |
| Stroke - CVA | 04 | 004 | 50 | F | 105 |
| Acute but limited stroke | 05 | 032 | 72 | M | 248 |
| Subarachnoid hemorrhage | 08 | 019 | 50 | M | 216 |
| Chronic post-anoxia (vegetative state) | 05 | 051 | 37 | F | 446 |
| Binswanger disease | 10 | 014 | 74 | M | 347 |
| Vasculitis | 10 | 009 | 57 | F | 1250 |
| Cerebral ischemia | 10 | 005 | 80 | M | 273 |
| Ischemia | 10 | 006 | 71 | F | 574 |
| Superior sagittal sinus thrombosis | 01 | 023 | 41 | M | 773 |

| **ORGANISMS** | | | | | |
|---|---|---|---|---|---|
| Cryptococcal meningitis | 05 | 033 | 29 | M | 1143 |
| Neuroborreliose | 05 | 045 | 16 | M | 141 |
| Chronic meningitis, unknown cause | 05 | 046 | 34 | F | 452 |
| Meningococcal meningitis | 05 | 049 | 24 | M | 37 |
| Bacterial meningitis | 05 | 059 | 73 | F | 446 |
| Neuro aids | 05 | 060 | 45 | F | 237 |
| Pneumococcal meningitis | 05 | 063 | 70 | M | 111 |
| Bacterial meningitis | 06 | 026 | 25 | M | 1250 |
| Bacterial meningitis | 06 | 027 | 19 | M | 37 |
| TBC meningitis | 06 | 028 | 54 | M | 720 |
| Bacterial meningitis | 06 | 029 | 23 | M | 37 |
| Bacterial meningitis | 06 | 030 | 28 | M | 88 |
| Viral meningitis | 06 | 037 | 82 | M | 314 |
| Bacterial meningitis | 08 | 018 | 49 | F | 205 |
| Viral meningitis | 08 | 029 | 29 | F | 170 |
| | | | | | |

| **CONTROLS** | | | | | |
|---|---|---|---|---|---|
| Muscle weakness and hysterical | 01 | 020 | 39 | F | 282 |
| conversion | | | | | |
| Facial palsy (peripheral nerve | 05 | 041 | 44 | M | 141 |
| disease, normal CSF | | | | | |
| Tension (psychogenic) headache | 05 | 031 | 49 | F | 37 |
| Psychogenic headache and neurosis | 05 | 036 | 56 | F | 131 |
| Psychogenic signs and symptoms | 05 | 040 | 48 | F | 220 |
| Major hysteria | 05 | 042 | 47 | F | 471 |
| Lumbar disc prolaps and sciatica | 05 | 047 | 74 | M | 195 |
| Control (associated Sjogren disease) | 05 | 065 | 52 | F | 107 |
| Control (neurosis) | 05 | 079 | 40 | F | 90 |
| Depression | 10 | 008 | 59 | F | 201 |
| Depression | 10 | 010 | 69 | F | 139 |
| Myalgia, myositis | 04 | 003 | 31 | M | 37 |
| Myalgia, myositis | 04 | 005 | 35 | F | 435 |
| Malaise, fatigue | 04 | 006 | 50 | M | 98 |
| Neck pain, vertigo | 08 | 011 | 39 | F | 192 |
| Headache | 08 | 017 | 49 | M | 113 |
| Depression | 08 | 021 | 57 | M | 361 |
| Headache, strabismus convergens | 08 | 033 | 32 | F | 239 |
| Bell's palsy | 06 | 031 | 38 | M | 90 |
| Bell's palsy | 06 | 032 | 22 | F | 131 |
| Bell's palsy | 06 | 033 | 73 | F | 231 |
| Bell's palsy | 06 | 034 | 74 | F | 144 |
| Carpal tunnel syndrome | 06 | 035 | 71 | M | 190 |
| Carpal tunnel syndrome | 06 | 036 | 90 | M | 665 |

| | | | | | |
|---|---|---|---|---|---|
| ^{a}01: Prof. P. Cras, University Hospital, Neurology, Antwerp, Belgium and Dr. A. Daniels, UIA, Laboratory of Neurobiology, Antwerp, Belgium; 04: Dr. P.D. Mehta, Institute Basic Research, Staten Island, NY, USA; 05: Dr. A. Ivanoiu, UCL St-Luc, Laboratory of Neurochemistry, Brussels, Belgium; 06: Prof. P.P. De Deyn, UIA, Laboratory of Neurochemistry, Antwerp, Belgium; 08: Dr. C. Bancher, Lainz Hospital, Neurology, Vienna, Austria; 10: Dr. J. Wiltfang, Georg-August University, Gerontopsychiatry, Göttingen, Germany. | | | | | |
| ^{b}F: female; M: male. | | | | | |
| ^{c}In cases where multiple factors may contribute to the damage, only the factor supposed to be | | | | | |

most relevant is given.

### FIGURE LEGENDS

**Figure 1.** Tau values at diagnosis, before any treatment was given: 1. Control children; 2. AML; 3. AML-CNS+; 4. CML; 5. MDS; 6. B-NHL; 7. Non-B-ALL; 8. Non-B-ALL CNS+; 9. VHR non-B-ALL.
**Figure 2.** CSF-tau levels in seven patients after acute ischemic stroke. The CSF samples were collected at income (day 0-1), day 2-3, day 7-8, day 21-22 (3 weeks) and day 90-110 (3 months).
**Figure 3.** CSF-tau levels at the time of maximal release in relation to the size of the infarction as measured by CT scan.

### EXAMPLES

### Example 1: Increased tau levels in children with leukemia

To evaluate the influence of chemotherapy on neuronal damage, a longitudinal study was conducted, involving 65 children with leukemia (aged 2 to 16 years) without measurable central nervous system involvement, and treated according to standard procedures. A total of 377 CSF samples were analysed. Before each injection, a small volume of fluid was sampled for routine laboratory analysis and the leftover was used in our study. These children were being diagnosed and then treated for their leukemia at the University Hospital of Leuven, Belgium. Tau protein in cerebrospinal fluid was assessed using the INNOTEST hTAU Antigen (Innogenetics, Gent, Belgium).

For all children suspected to have leukemia, a lumbar puncture was performed before the start of the treatment to detect the possible presence of leukemic cells, indicative of central nervous system invasion. The tau levels measured at that time, and thus before treatment, would serve as the control level to compare chemotherapy-induced changes in the levels of tau.

We observed that some children with leukemia already had very high tau levels at diagnosis in spite of the fact that no leukemic cells were detected in the central nervous system. These children constitute a new risk group having brain invasion or leukemia-induced CNS damage, which cannot always be found using current diagnostic procedures (imaging of the brain, lumbar puncture, eye fundoscopy). This was further supported by the increased tau levels seen in one patient having leukemia with proven cellular invasion into the brain (malignant cells in the cerebrospinal fluid).

### Example 2: Increased tau levels in patients with leukemia before treatment

### 1. Subjects

Between August 1996 and June 1999, 510 samples of CSF were taken from 82 children being treated for cancer at the Pediatric Hemato-oncology Department of the Catholic University of Leuven, Belgium. CSF samples were only taken in the course of scheduled lumbar punctions (LPs) for staging or treatment for malignancy.

Three groups of patients with hematological malignancies were studied. The largest group consisted of 48 patients with non-B-ALL, treated according to the EORTC protocol 58881 (table 1). Of these children, 20 had CD10(+) blasts (or common ALL), from whom two patients had also Down syndrome (DS) and 1 patient had the Brachmann-de Lange syndrome; 6 patients had common B-cell blasts, 2 patients had common T-cell blasts, 2 patients had pro-B-cell blasts, 9 patients had pre-B-cell blasts, and 9 patients had T-cell blasts. Fourty-two children had leukemia, 6 patients had non-Hodgkin's lymphoma stage II (1 patient), Stage III (4 patients) or Stage IV (1 patient). One patient had overt CNS involvement (CNS+), defined according to the study protocol with malignant cells in the CSF. Five patients were considered as very high risk (VHR) patients according to the criteria defined in the protocol (2 patients with t(9;22) and 3 patients with corticoid-resistance). As the first part of the induction treatment was similar to the other patients, they were included in the analyses according to the induction chemotherapy. Twenty-eight of the patients within non-B-ALL could be followed longitudinally during their treatment period.

A second group of patients included 10 B-cell non-Hodgkin's lymphoma patients, treated according to the United Kingdom Children Cancers group (UKCCSG 9602) NHL protocol (table 2). Five patients had B-cell lymphomas, 3 patients had Burkitt's lymphoma, and 2 patients had anaplastic large cell lymphoma (ALCL). All patients were treated with the same protocol, except one patient who had B-cell leukemia. Six of these patients were studied longitudinally. A third patient group consisted of 9 children with Acute Myeloid Leukemia - myelodysplasia (AML - MDS), of which 2 patients had CNS involvement and two patients had Down syndrome. There were 3 patients with M0, and 1 patient each with M1, M2, M5a or M7 phenotype. Two patients had MDS, of which one had developed AML and was treated with chemotherapy. All these patients, except one MDS patient, were treated according to the EORTC 58921 protocol (table 3) and 7 patients were followed longitudinally.

The other patients consisted of a heterogeneous group of children (n = 9) in which for clinical reasons an LP was performed. This group includes 3 children with medulloblastoma (staging), 2 children with rhabdomyosarcoma (staging), 1 child with Langerhans cell histiocytosis (LCH, staging), ganglioglioma (staging), germinoma (staging), and retinoblastoma with CNS metastasis (staging and follow up). The control group consisted of 4 children from whom CSF was taken as part of the routine control for possible viral or bacterial infections, but with negative results. One patient with a localized retinoblastoma (staging) and one screened for familial hemophagocytic lymphohistiocytosis (HLH) were also included in the control group. The nearest relatives of the patients gave oral informed consent for participation in the study.

### 2. Methods

**Liquor sampling.** Lumbar punctures were performed just prior to the IT administration of therapeutics. Five ml of CSF was collected in different polypropylene tubes. One sample was centrifuged immediately at 1500 rpm for 2 minutes to eliminate cells and other insoluble material. The supernatant was stored at -70°C for subsequent analysis. The number of freeze/thaw cycles was restricted to a minimum.

**Measurement of tau in the CSF.** All biochemical analyses for the detection of CSF-tau were made without knowledge of the clinical diagnosis. Potentially confounding factors, such as the number of freeze/thaw cycles, recipient type, and volume of sample per assay were standardized for the whole study protocol. CSF-tau levels were determined using a sandwich ELISA (INNOTEST hTAU Antigen, Innogenetics, Gent, Belgium), that measured total tau (both normal and hyperphosphorylated tau). Samples were analyzed firstly alone, at the time of each LP Afterwards, all samples derived from one patient were analyzed again on one immunoplate. The correlation coefficient between the results from the first and the second approach for a set of 104 samples was 0.901 (95%CI: 0.856 - 0.933). The increases of CSF-tau described in the paper were excluded to be general increases of proteins in the liquor.

### 3. Results

Normal upper limit levels for tau were firstly determined on CSF samples from the 6 control children. The mean CSF-tau value was 106.2 pg/ml (95%CI = 34.3-178.0). Arbitrary cut-off normal value was considered as 312 pg/ml (mean + 3 SD), which is in the range of values observed in adults (Hulstaert et al., 1999). Moreover, we did not find a correlation between the CSF-tau values at diagnosis and the age of the children (Pearson r = -0.161, CI95% = -0.3914 - 0.08836, n = 64), suggesting that age has no influence on CSF-tau levels. Clearly pathological CSF-tau values can be considered as above 500 pg/ml.

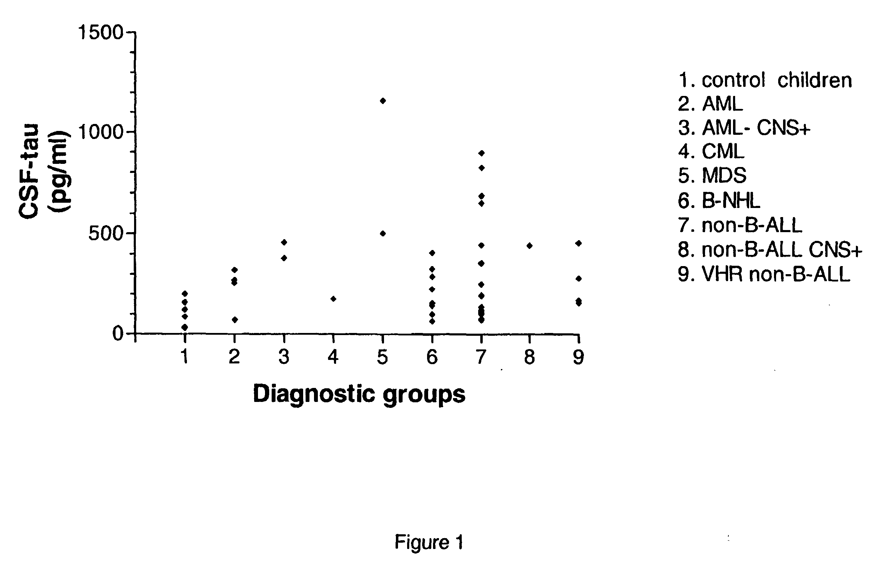

Tau levels at diagnosis were analyzed for each subgroup of patients (Fig. 1). There was no obvious difference for the tau levels in patients with and without Dow's syndrome (not shown). The two patients with overt CNS invasion (CNS+) had levels of CSF-tau at diagnosis above 312 pg/ml. However, the 2 children with MDS, 7/28 children with non-B-ALL, 1/4 non-B-ALL patients with very high risk criteria, 1/5 patients with AML, and 2/8 patients with B-cell NHL had a level of tau above 312 pg/ml, while using classical diagnostic procedures, CNS invasion was not detected. Three patients with risen intracranial pressure (medulloblastoma), and one patient with germinoma, from which CSF was taken for staging, had high CSF-tau concentrations (823,1397,1500 and 442 pg/ml), in contrast to one patient with grade I astrocytoma who had a normal CSF-tau level (97 pg/ml). One patient with LCH had a normal CSF-tau level of 112 pg/ml. Two patients with rhabdomyosarcoma could be analyzed at diagnosis: one patient with stage I disease had a CSF-tau level of 279 pg/ml, the other patient with stage IV disease had a level of 320 pg/ml. One patient with retinoblastoma and CNS involvement had a CSF-tau level of 1800 pg/ml.

For 33 patients with non-B-ALL, CSF-tau levels at diagnosis did not correlate with tumor burden, as reflected by the white blood cell count (Pearson r = 0.04575, CD95%: -0.3024 - 0.3831) or serum LDH (Pearson r = -0.03002, CI95%: -0.3696 - 0.3166). In patients with B-NHL, there was no significant correlation between the LDH level and CSF-tau (Pearson r = -0.3723, CD95%: - 0.8532 - 0.4507).

### Example 3: Use CSF-tau as a marker for early detection of possible CNS damage caused by stroke

### 1. Subject

Seven patients, 3 men and 4 women, 63-81 years old (mean SD, 70.7±7.2 years) with cerebral infarctions admitted to the Unit of Neurology, Sahlgren's University Hospital, Göteborg, Sweden, were incorporated in the study. All patients were included within 72 h of stroke onset.

### 2. Methods

CSF samples were collected using lumbar puncture. 12 ml was collected and frozen in 0.5 ml aliquots at -80°C until analyzed. CSF samples were collected at income (day 0-1), day 2-3, day 7-8, day 21-22 (3 weeks) and day 90-110 (3 months). CSF-tau levels were determined using a sandwich ELISA (INNOTEST hTAU Antigen, Innogenetics, Gent, Belgium), that measured total tau (both normal and hyperphosphorylated tau).

The extent of brain damage was examined with computerized tomography (CT) of the brain during the first day of admittance. Clinical the patients were also examined using the modified Scandinavian Stroke Scale Index (SSI; Scandinavian Stroke Study Group, 1985) at onset of stroke and 3 months later for the degree of disability with the Bartel Index (BI; Mohoney and Barthel, 1965).

### 3. Results

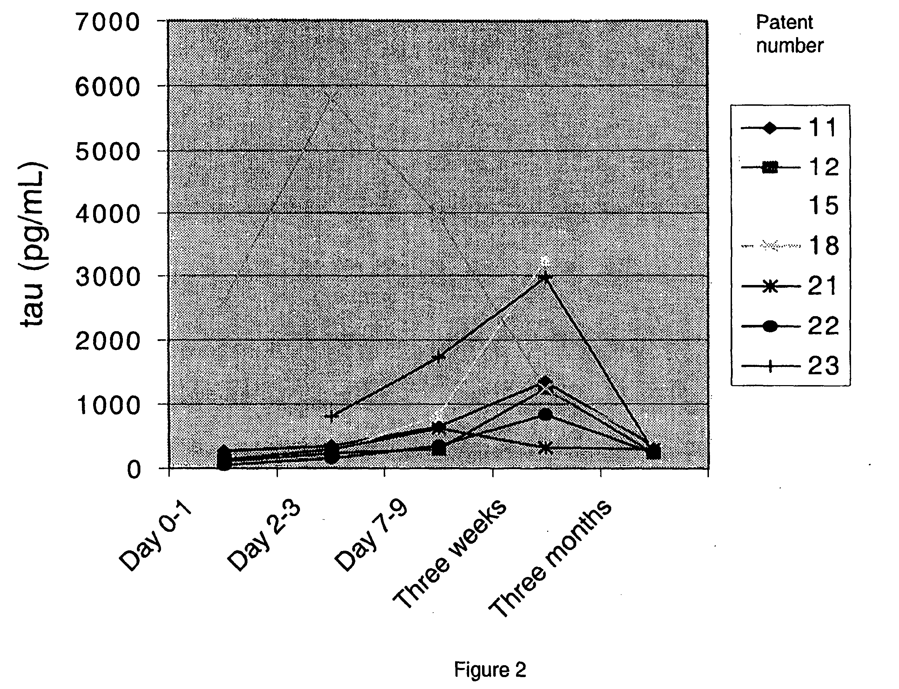

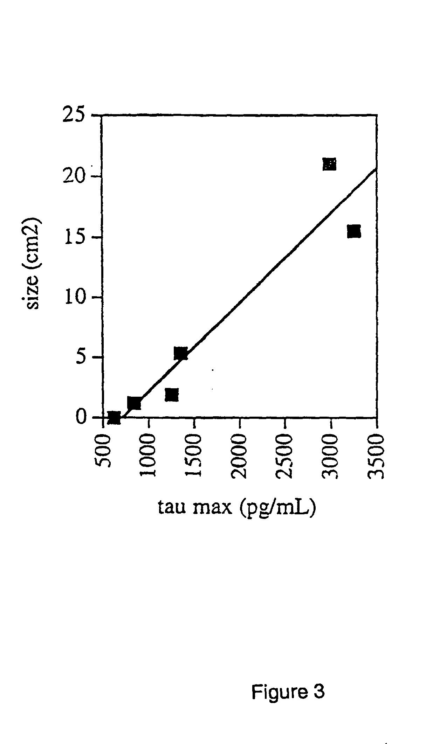

CSF-tau showed a marked increase after acute stroke, with a peak after 1-3 weeks and return to normal after 3 months (Fig 2). There was also a correlation between CSF-tau levels and the size of the infarction as measured by CT scan (Fig 3). These results indicate that CSF-tau reflects neuronal damage and degeneration and the level in the CSF depends on the amount of damaged nerve cells. In this study no correlation was found between the clinical data (SSI or BI) and CSF-tau, probably due to small number of patients.

### Example 4: Use of tau as a marker for early detection of possible CNS damage caused by different damaging agents

### 1. Subjects

A multicentre study was carried out at 8 European and 2 U.S. university centres involved in CSF research, based on residual CSF archived at the centres for research purposes. CSF samples of patients with a broad range of different neurological conditions were included in order to get a general idea about the specificity of the tau marker changes in a variety of pathologies involving the CNS. Neurological controls consisted of subjects without obvious CNS damage (table 4).

The study was conducted in accordance with local clinical research regulations. If required additional local Ethics Committee or Institutional Review Board approval was obtained by the investigator prior to the start of the study.

### 2. Methods

CSF samples were collected using lumbar puncture (LP). Only CSF samples containing less than 500 red blood cells per µl were included in the study. The CSF samples were centrifuged at 2000 g for 10 minutes within 4 hours after LP and kept frozen without thawing CSF samples from centre 01 had undergone an additional freeze-thaw cycle before analysis. The concentration of total tau comprising normal tau and paired helical filament-tau was measured at the centres using a sandwich ELISA technique (INNOTEST hTAU-Antigen, Innogenetics N.V.).

### 3. CSF-tau levels in patients with possible CNS damage compared to CSF-tau levels in neurological control subjects

Tau levels in the CSF samples of patients with possible CNS damage caused by space occupying lesions, by invasion or metastasis, by bleeding, infarction or ischemia or by organisms and tau levels in CSF samples of control subjects are shown in table. The group of patients that had possibly contracted CNS damage by space occupying lesions, by invasion or metastasis, by bleeding, by infarction or ischemia; or by organisms showed an overall higher tau level than the neurological control patients (p = 0.022, two sided Mann Whitney test).

### REFERENCES

Andreadis A., Brown W., Kosik K. (1992) Structure and novel exons of the human tau gene. Biochem. 31: 10626-10633.
Arbit E., Cheung N.K., Yeh S.D., Daghighian F. ,Shang J.J., Cordon-Cardo C., Pentlow K., Canete A., Finn R., Larson S.M. (1995) Quantitative studies of monoclonal antibody targeting to disialogangliosid GD2 in human brain tumours. Eur. J. Nucl. Med. 22: 419-426.
Bramblett G., Trojanowski J., Lee V. (1992) Regions with abundant neurofibrillary pathology in human brain exhibit a selective reduction in levels of binding-competent tau and accumulation of abnormal tau isoforms (A68 proteins). Lab. Invest. 66: 212-222.
Bramblett G., Goedert M., Jakes R., Merrick S., Trojanowski J., Lee V. (1993) The abnormal phosphorylation of tau at Ser396 in Alzheimer's disease recapitulates phosphorylation during development and contributes to reduced microtubule binding. Neuron. 10: 1089-1099.
Delacourte A., Flament S., Dibe E., Hublau P., Sablonniere B., Hemon B., Sherrer V., Defossez A. (1990) Pathological proteins Tau64 and 69 are specifically expressed in the somatodendritic domain of the degenerating cortical neurons during Alzheimer's disease. Acta Neuropathol. 80: 111-117.
Flament S., Delacourte A. (1990) Tau Marker? Nature 346: 6279.
Ghanbari H., Kozuk T., Miller B., Riesing S. (1990) A sandwich enzyme immunoassay for detecting and measuring Alzheimer's disease-associated proteins in human brain tissue. J: Clin. Laboratory Anal. 4: 189-192.
Goedert M., Spillantini M., Jakes R., Rutherford D., Crowther R. (1989) Multiple isoforms of human microtubule-associated protein tau: sequences and localisation in neurofibrillary tangles of Alzheimer's disease. Neuron. 3: 519-526.
Goedert M., Cohen E., Jakes R., Cohen P. (1992) p42 Map kinase phosphorylation sites in microtubule-associated protein tau one dephosphorylated by protein phosphatase 2Al: implications for Alzheimer's disease. FEBS Lett. 312: 95-99.
Goedert M., Jakes R., Crowther R., Six J., Lübke U., Vandermeeren M., Cras P., Trojanowski J.Q., Lee V. (1993) The abnormal phosphorylation of tau protein at serine 202 in Alzheimer's disease recapitulates phosphorylation during development. Proc. Natl. Acad. Sci. (USA) 90: 5066-5070.
Greenberg S., Davies P. (1990) A preparation of Alzheimer paired helical filaments that displays distinct tau proteins by polyacrylamide gel electrophoresis. Proc. Natl. Acad. Sci. USA 87: 5827-5831.
Harrington C., Mukaetova E., Hills R., Edwards P., Montejo de Garcini E., Novak M., Wischik C. (1991) Measurement of distinct immunochemical presentations of tau protein in Alzheimer's disease. Proc. Natl. Acad. Sci. (USA) 88: 5842-5846.
Hasegawa M., Morishima-Kawashima M., Takio K., Suzuki M., Titani K., Ihara Y. (1992) Protein sequence and mass spectrometric analyses of tau in Alzheimer's disease brain. J. Biol. Chem 267: 17047-17054.
Himmler A. (1989) Structure ofthe bovine tau gene: alternatively spliced transcripts. Mol. Cell Biol. 9(4): 1389-96.
Huang Q., He G., Lan Q., Li X., Qian Z. Chen J. Lu Z., Du Z. (1996) Target imaging diagnosis of human brain glioma. Clinical analysis of 40 cases. Nucl. Med. Commun. 17: 311-316.
Hulstaert F., Blennow K., Ivanoiu A., Schoonderwaldt H.C., Riemenschneider M., De Deyn P.P., Bancher C., Cras P., Wiltfang J., Mehta P.D., Iqbal K., Pottel H., Vanmechelen E., Vanderstichele H. (1999) Improved discrimination of AD patients using β-amyloid₍₁₋₄₂₎ and tau levels in CSF. Neurology 52: 1555-1562.
Iqbal K., Grundke-Iqbal I., Smith A., George L., Tung Y., Zaidi T. (1989) Identification and localisation of a Tau peptide to paired helical filaments of Alzheimer's disease. Proc. Natl. Acad. Sci. (USA) 86: 5646-5650.
Ksiezak-Reding H., Liu W.K., Yen S.H. (1992) Brain Res. 597: 209-219.
Lee V., Balin B., Otvos L., Trojanowski J. (1991) A68: a major subunit of paired helical filaments and derivatized forms of normal tau. Science 251(4994): 675-8.
Lewis S., Wang D., Cowan N. (1988) Microtubule-associated protein MAP2 shares a microtubule binding motif with Tau protein. Science 242: 936-939.
Mariani G., Lasku A., Pau A., Villa G., Motta C., Calcagno G., Taddei G.Z., Castellani P., Syrigos K., Dorcaratto A., Epenetos A.A., Zardi L., Viale G.A. (1997) A pilot pharmacokinetic and immunoscintigraphic study with the technetium-99m labelled monoclonal antibody BC-1 directed against oncofetal fibronectin in patients with brain tumours. Cancer 15: 2484-2489.
Mohoney F.I., Barthel D.W. (1965) Functional evaluation: The Barthel Index. Md. State. Med. J. 24: 61-69.
Raichle M.E. (1998) Behind the scenes of functional brain imaging: A historical and physiological perspective. Proc. Natl. Acad. Sci. USA 95: 765-772.
Sandrock D., Verheggen R., Helwig A.T., Munz D.L., Markakis E., Emrich D. (1996) Immunoscintigraphy for the detection of brain abscesses. Nucl. Med. Commun. 17: 311-316.
Scandinavian Stroke Study Group (1985) Multicenter trial of hemodilution in ischemic stroke: Background and study protocol. Stroke 16: 373-403.
Selden S., Pollard T. (1983) Phosphorylation ofmicrotubule-associated proteins regulates their interaction with actin filaments. J. Biol. Chem. 258(11): 7064-71.
Tamada K., Fujinaga S., Watanabe R., Yamashita R., Takeuchi Y., Osano M. (1995) Specific deposition of passively transferred monoclonal antibodies against herpes simplex virus type 1 in rat brain infected with the virus. Microbiol-Immunol. 39: 861-871.
Vandermeeren M., Mercken M., Vanmechelen E., Six J., Van de Voorde A., Martin J., Cras P. (1993) Detection of tau proteins in normal and Alzheimer's disease fluid with a sensitive sandwich enzyme linked assay J. Neurochem. 61: 1828-1834.
Van de Voorde A., Vanmechelen E., Vandermeeren M., Dessaint F., Beeckman W., Cras P. (1995) Detection of tau in cerebrospinal fluid. Research Advances in Alzheimer's disease and related disorders, Ed. Ibqal, Mortimer, Winblad & Wisniewski, John Wiley & Sons Ltd.
Wakabayashi T., Yoshida J., Okada H., Sugita K., Itoh K., Tadokoro M., Ohshima M. (1995) Radioimaging of human glioma by indium-11 labelled G-22 anti-glioma monoclonal antibody. Noshuyo-Byori 12: 105-110.
Wilson J.D., Braunwald E., Isselbacher K.J., Petersdorf R.G., Martin J.B., Fauci A.S., Root R.K. (1991) Harrison's Principles ofInternal Medicine, 12th Edition, McGraw-Hill Inc, NY, USA.

## Claims

1. A method for the in vitro detection and/or quantification of CNS damage in an individual before said CNS damage is detectable by standard diagnostic methods based on brain imaging, lumbar puncture or eye fundoscopy, said CNS damage being caused by a primary brain tumor, benign or malignant, brain metastasis, parasite derived cysts, hydrocephalus and/or subdural haematoma, by invasion or metastasis of the CNS, by organisms, by anoxia or ischemia, by a chemical agent which is gene therapy, pharmaceuticals, chemotherapy, or exposure to chemical compounds, by physical agents or by a combination of these mechanisms, said method comprising the step of determining the level of tau in a CSF sample from said individual and comparing it to the level of tau in a CSF sample from control healthy individuals, whereby a level of tau above a cut-off value, determined based on the level of tau in a CSF sample from control healthy individuals, is an indication of the individual having brain damage.

2. A method according to claim 1 in which the invasion or metastasis of the CNS is by leukemia, lymphoma or breast cancer.

3. A method according to claim 1 in which the organisms are bacteria or viruses causing encephalitis or meningitis.

4. A method according to claim 1 in which the anoxia or ischemia is caused by stroke, by cerebral infarction, by cerebral hemorrhage, by thrombosis, by perinatal asphyxia, by Binswanger disease or by vasculitis.

5. A method according to claim 1 in which the physical agent is a trauma, stroke, intracranial pressure or radiation.

6. A method according to any of claims 1 to 5 in which CNS damage is detected and/or quantified in order to evaluate the effect of a certain treatment on said CNS damage.

7. A method according to any of claims 1 to 6, further **characterized** that the level of tau is determined by use of an antibody that recognizes total tau.

## Patentansprüche

1. Verfahren zur In-vitro-Erfassung und/oder - Quantifizierung einer ZNS-Schädigung in einem Individuum, bevor sich die ZNS-Schädigung durch Standard-Diagnoseverfahren, die auf Gehirn-Bildgebung, Lumbalpunktur oder Augenfundoskopie beruhen, nachweisen lässt, wobei die ZNS-Schädigung durch einen primären gutartigen oder bösartigen Gehirntumor, Gehirn-Metastase, von Parasiten stammenden Cysten, Hydrocephalus und/oder subdurale Hämatome, durch Befall oder Metastase des ZNS, durch Organismen, durch Anoxie oder Ischämie, durch ein chemisches Mittel, bei dem es sich um ein Gentherapeutikum, Pharmazeutikum, Chemotherapeutikum handelt, oder durch Aussetzen gegenüber chemischen Verbindungen, durch physikalische Mittel oder durch eine Kombination dieser Mechanismen verursacht wird, wobei das Verfahren das Bestimmen des tau-Spiegels in einer CSF-Probe aus dem Individuum und das Vergleichen mit dem tau-Spiegel in einer CSF-Probe aus gesunden Kontrollindividuen umfasst, wobei ein tau-Spiegel oberhalb eines Ausschlusswertes, bestimmt auf der Basis des tau-Spiegels in einer CSF-Probe aus gesunden Kontrollindividuen, ein Anzeichen dafür ist, dass das Individuum eine Gehirnschädigung hat.

2. Verfahren nach Anspruch 1, wobei der Befall oder die Metastase des ZNS durch Leukämie, Lymphom oder Brustkrebs erfolgt.

3. Verfahren nach Anspruch 1, wobei die Organismen Bakterien oder Viren sind, die Enzephalitis oder Meningitis verursachen.

4. Verfahren nach Anspruch 1, wobei die Anoxie oder Ischämie durch einen Schlaganfall, Hirninfakt, cerebrale Hämorrhagie, Thrombose, durch perinatale Asphyxie, Binswanger-Erkrankung oder Vasculitis verursacht wird.

5. Verfahren nach Anspruch 1, wobei das physikalische Mittel ein Trauma, Schlaganfall, Intrakranialdruck oder Strahlung ist.

6. Verfahren nach einem der Ansprüche 1 bis 5, wobei die ZNS-Schädigung erfasst und/oder quantifiziert wird, damit die Wirkung einer bestimmten Behandlung auf die ZNS-Schädigung bewertet wird.

7. Verfahren nach einem der Ansprüche 1 bis 6, weiterhin **dadurch gekennzeichnet, dass** der tau-Spiegel durch die Verwendung eines Antikörpers, der Gesamt-tau erkennt, bestimmt wird.

## Revendications

1. Procédé pour la détection et/ou la quantification *in vitro* d'une lésion du SNC chez un individu avant que ladite lésion du SNC ne soit détectable par des procédés de diagnostic standard basés sur une imagerie du cerveau, une ponction lombaire ou une fondoscopie des yeux, ladite lésion du SNC étant provoquée par : une tumeur primaire du cerveau, bénigne ou maligne ; une métastase cérébrale ; des kystes dérivés de parasites ; un hématome d'hydrocéphalie et/ou sous-dural ; par une invasion ou une métastase du SNC ; par des organismes ; par une anoxie ou une ischémie ; par un agent chimique qui est une thérapie génique, des substances pharmaceutiques, une chimiothérapie ou une exposition à des composés chimiques ; par des agents physiques ; ou par une combinaison de ces mécanismes, ledit procédé comprenant l'étape consistant à déterminer le taux de protéine tau dans un échantillon de CSF provenant dudit individu et en le comparant avec le taux de protéine tau dans un échantillon de CSF provenant d'individus témoins sains, moyennant quoi un taux de protéine tau se trouvant au-dessus d'une valeur limite, déterminée sur la base du taux de protéine tau dans un échantillon de CSF provenant d'individus témoins sains, est une indication d'une lésion cérébrale chez un individu.

2. Procédé selon la revendication 1, dans lequel l'invasion ou la métastase du SNC se fait par une leucémie, un lymphome ou un cancer du sein.

3. Procédé selon la revendication 1, dans lequel les organismes sont des bactéries ou des virus causant une encéphalite ou une méningite.

4. Procédé selon la revendication 1, dans lequel l'anoxie ou l'ischémie est causée par : un ictus, un infarctus du cerveau, une hémorragie cérébrale, une thrombose, une asphyxie périnatale, une démence pré-sénile du type Binswanger ou une vascularite.

5. Procédé selon la revendication 1, dans lequel l'agent physique est un traumatisme, un ictus, une pression intra-crânienne ou une irradiation.

6. Procédé selon l'une quelconque des revendications 1 à 5, dans lequel la lésion du SNC est détectée et/ou quantifiée dans le but d'évaluer l'effet d'un certain traitement sur ladite lésion du SNC.

7. Procédé selon l'une quelconque des revendications 1 à 6, **caractérisé en outre en ce que** le taux de protéine tau est déterminé au moyen de l'utilisation d'un anticorps qui reconnaît la protéine tau totale.
